# EUROPEAN PATENT APPLICATION

(11) **EP 1 380 647 A1**
(43) Date of publication of application: **14.01.2004**
(21) Application number: 02702724.2
(22) Date of filing: 04.03.2002
(51) Int. Cl.: C12N 15/53, C12N 9/02, C12N 5/10, A01H 5/00

(54) **DEOXYMUGINEIC ACID SYNTHASE AND GENE THEREOF**

(30) Priority: 23.03.2001 JP 2001086162
(71) Applicant: JAPAN SCIENCE AND TECHNOLOGY CORPORATION, Kawaguchi-shi, Saitama 332-0012 (JP)
(72) Inventor: NISHIZAWA, Naoko, Bunkyo-ku, Tokyo 113-0001 (JP); MORI, Satoshi, Bunkyo-ku, Tokyo 113-0033 (JP); NEGISHI, Takashi, Kawaguchi-shi, Saitama 333-0866 (JP)
(74) Representative: Cresswell, Thomas Anthony
(86) International application number: PCT/JP2002/001940
(87) International publication number: WO 2002/077240

(57) **Abstract**

A reductase reducing keto-nicotianamine into 2'-deoxymugineic acid in the course of the biosynthesis of mugineic. acids participating in the iron-acquisition mechanism in plants; a gene encoding this enzyme; a plant having a enhanced tolerance to iron-deficiency owing to the above gene transferred thereinto; and enzyme kits and gene kits for constructing a plant having a enhanced tolerance to iron-deficiency which comprise enzymes for biosynthesizing mugineic acids participating in the iron-acquisition mechanism in plants or genes encoding these enzymes.

## Description

### Technical Field

The present invention relates to a reductase reducing keto-nicotianamine into 2'-deoxymugineic acid in the course of the biosynthesis of mugineic acids participating in the iron-acquisition mechanism in plants; a gene encoding this enzyme; and a plant having an enhanced tolerance to iron-deficiency owing to the above gene transferred thereinto. Also, the invention relates to enzyme kits and gene kits constructing a plant having an enhanced tolerance to iron-deficiency which comprise enzymes for biosynthesizing mugineic acids participating in the iron-acquisition mechanism in plants or genes encoding this enzyme.

### Background Art

Iron is the fourth abundant element in an existing quantity on the earth crust and is the essential element for plants. Iron is present as an insoluble form of trivalent iron in a common aerobic condition where oxygen exists, and plants can not absorb iron present as the insoluble form. Particularly, where limestone is abundant, soil becomes alkali, many plants can not absorb iron in the insoluble form and can not take it resulting in being blighted due to iron-deficiency.

The plants have acquired the mechanism for absorption of iron by solubilizing trivalent iron in the insoluble form in order to absorb and utilize iron which is an essential element for the plants. Two types of processes referred to as "Strategy I" and "Strategy II" have been known as the mechanisms acquired to solubilize trivalent iron in the insoluble form and absorb it.

In the mechanism referred to as "Strategy I", Fe³⁺ in the insoluble form is solubilized by releasing protons from roots to lower pH in a rizosphere, and further is reduced to Fe²⁺ by an iron reducing enzyme present on the surface of root followed by being incorporated into cells by a transporter of divalent iron. This iron acquisition mechanism is employed by dicotyledonous plants and monocotyledonous plants other than Graminaceous plants. On the other hand, the Graminaceous plants absorb iron by the highly dextrous mechanism where mugineic acids which are natural iron chelators collectively referred to as phytosiderophores are secreted from roots, and trivalent iron in the insoluble form in soil is solubilized as an "iron-mugineic acid" chelate to absorb from the roots in the form of the chelate. This iron acquisition mechanism of Graminaceous plants is referred to as "Strategy II".

Mugineic acid (MA) is the compound represented by the following formula: 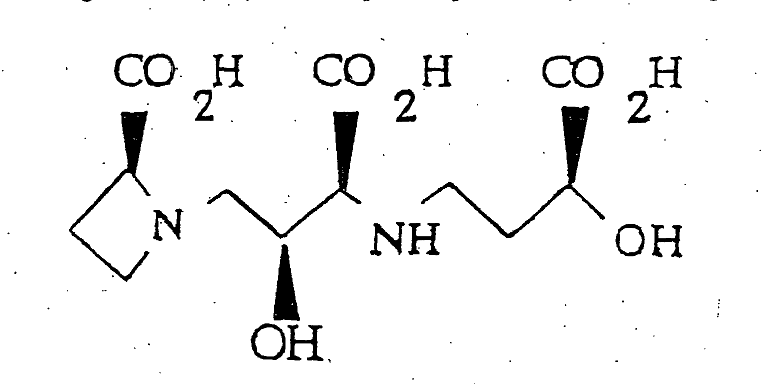 having 4-membered ring containing nitrogen atoms, and this mugineic acid and five types of derivative thereof have been known as natural iron chelators collectively referred to as phytosiderophores which form the chelate with trivalent iron. These five derivatives are deoxymugineic acid (DMA) which is a precursor of mugineic acid and lacks a hydroxy group, avenic acid (AVA) which is a open-ring form of deoxymugineic acid (DMA), 3-hydroxymugineic acid (HMA) and 3-epi-hydroxymugineic acid ((epi)-HMA) where a hydroxy group is bound to an azetidine ring of mugineic acid, and disticomic acid which is a open-ring form of 3-hydroxymugineic acid.

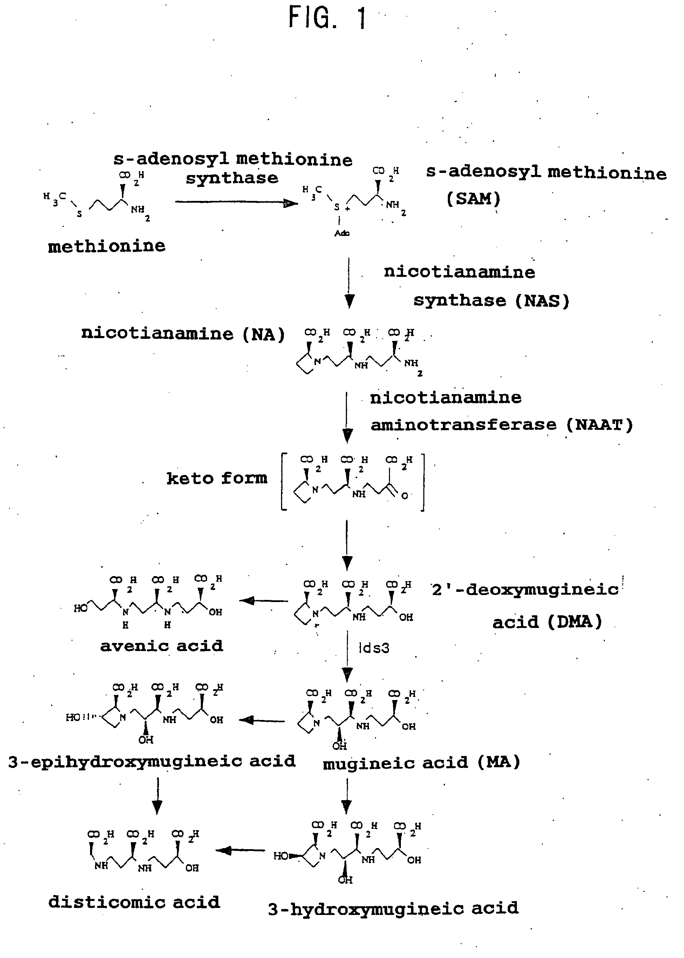

The chemical structure formulae of these mugineic acid and derivatives thereof and their biosynthetic pathways are shown in Fig. 1. First, S-adenosylmethionine (SAM) is produced from methionine by S-adenosylmethionine synthase. Then, three molecules of S-adenosylmethionine are bound by nicotianamine synthase to generate one molecular nicotianamine (NA). Nicotianamine amino group transferase converts the generated nicotianamine into 3"-keto body, and subsequently some reductase converts it into 2'-deoxymugineic acid (DMA). This is further hydrolyzed to become the other mugineic acid derivatives including mugineic acid (MA) (Mori and Nishizawa, Plant Cell Physiol., 28:1081-1092, 1987; Shojima et al., Biol. Metals, 2:142-145, 1989; Shojima et al., Plant Physiol., 93:1497-1503, 1990).

The synthesis and secretion of mugineic acid are strongly induced by an iron-deficiency condition. There are 6 types of mugineic acids ever known. The type and amount of secretion of the mugineic acid is different depending on species of Graminaceous plants, and this difference is believed to be the difference of tolerance to iron-deficiency among species of Graminaceous plants.

Rice, maize and wheat secret DMA which is the first mugineic acid in the biosynthetic pathways of mugineic acids. Barley secrets DMA, MA, and further hydroxylated (epi)-HMA. Rye secrets DMA, MA and HMA. Oat secrets DMA and avenic acid (AVA). So far as being examined, Graminaceous plants which have the iron-acquisition mechanism by "Strategy II" all secret DMA, and it is thought that they have the biosynthetic pathway to DMA.

In the biosynthetic pathways of mugineic acids, the activities of nicotianamine synthase (NAS) which catalyzes the reaction from SAM to NA and of nicotianamine amino group transferase (NAAT) which catalyzes the reaction from NA to the keto body are strongly induced by the iron-deficiency treatment (Kanazaw, et al., ed J. Abadia, 37-41, .1995). Both of these enzymes were purified from roots of the iron-deficient barley and their genes were cloned (Takahashi, et al., Plant Physiol., 121:947-956, 1999; Higuchi, et al., Plant Physiol., 119:471-479, 1999).

IDS3 is a protein involved in the synthesis that converts DMA to MA. The Ids 3 gene is expressed most strongly by the iron-deficient treatment (Nakanishi, et al., Plant Cell Physiol., 34:401-410, 1993). Among Graminaceous plants, IDS3 protein is detected in barley and rye which secret mugineic acids including MA and its downstream intermediates in the biosynthetic pathway. In the experiments using wheat-barley chromosome addition strains in which each one chromosome of barley is added into wheat genome, MA is secreted only in the plant with the addition of the 4th chromosome of barley, and IDS3 is expressed only in the strain with the addition of the 4th chromosome, having led to presume that IDS3 might be mugineic acid synthase. And more recently, it has been found that MA is also secreted in addition to DMA by analyzing mugineic acids secreted by transformed rice having the transferred Ids 3 gene. Taken together, it has been concluded that IDS 3 is the mugineic acid synthase.

Also, the gene of SAM synthase which produces SAM from methionine has been isolated (Takizawa et al., J. Plant Nu 19(8-9):1189-1200, 1996), and almost of all enzymes involved in the pathways to biosynthesize mugineic acid (MA) from methionine have been elucidated. However, for the enzyme to synthesize DMA from the keto body, neither its protein nor gene have been purified and isolated yet although it has been known that it might be an NADH- or NADPH-dependent reductase.

The present invention aims to isolate and identify a reductase producing DMA from the keto body and a gene thereof, thereby elucidating the full picture of the enzymes involved in the biosynthesis of mugineic acids, and provides means to introduce the iron-acquisition mechanism into plants.

By the way, phenomena characteristic for the secretion of mugineic acid in the plant include circadian rhythm. The barley initiates the secretion of mugineic acid after the sunrise, decreases after 3 to 5 hours' peak, and ceases the secretion after the sunset (Takagi, et al., J. Plant Nu., 469-477, 1984). The fine structure of the barley root is changed due to iron-deficiency, and the increased number and size of special granules in tip cells of the root are observed. The granules appeared to be derived from rough-surfaced endoplasmic reticulum (rER) because libosome is on the surface and the membrane is thinner than that of Golgi. This granule is observed in the cortical cell, epidermal cell and root cap cell. This granule is present in an expanded state before the sunrise when mugineic acids are secreted, and is constricted along with the secretion of mugineic acids. Therefore, the granule has been presumed to be the synthetic and stored site of mugineic acids (Nishizawa, et al., J. Plant Nu., 15:695-713, 1987).

Thus, the localization of proteins (NAS, NAAT) involved in the biosynthetic pathway of mugineic acids was observed by a transient assay method using an immune staining method and green fluorescent protein (GFP) derived from jelly fish, and it has been shown that all proteins are localized in the special granules which increase in the barley roots with iron-deficiency (data is not published). Furthermore, it can be elucidated more clearly that the granule is the synthetic site of mugineic acids by identifying the reductase producing DMA from the keto body and demonstrating the localization thereof.

Also recently, the analysis of the transformed rice having the transferred naat-A has been carried out. In the analysis, it has been found that some rice grow in soil with iron-deficiency without developing chlorosis and that the amount of mugineic acid secretion is also increased. This is thought to be due to increasing the synthesized amount of the keto body by the introduction of naat-A and producing DMA by the endogenous reductase. However, in this case, the produced amounts of DMA and mugineic acid are limited by the amount of the endogenous reductase. Thus, it is believed that when naat-A and the gene of reductase are simultaneously transferred into rice, the rice having a more enhanced tolerance to iron-deficiency can be constructed than the transformed rice having only transferred naat-A.

Thus, in order to construct a plant having a more enhanced tolerance to iron-deficiency, it has been desired to demonstrate the full picture of the enzyme group biosynthesizing mugineic acids which are iron chelators collectively referred to as phytosiderophores in the iron acquisition mechanism.

### Disclosure of the Invention

The present invention aims to demonstrate the full picture of the enzyme group biosynthesizing mugineic acids which are iron chelators collectively referred to as phytosiderophores in the iron acquisition mechanism, and aims to isolate and identify a reductase producing DMA form the keto body and gene thereof for the above purpose.

Also, the present invention aims to demonstrate the full picture of enzyme group biosynthesizing mugineic acids in plants and to construct plants having more enhanced tolerance to iron-deficiency.

### Brief Description of the Drawings

Fig. 1 shows the biosynthetic pathways of mugineic acid and derivatives thereof in Graminae plants.
Fig. 2 is a photo in place of a drawing showing that three amplified fragments are obtained by PCR using degenerate primers.
Fig. 3 is photos in place of drawings showing the results of Northern blot analyses of shoot and root portions of barley in iron-deficient (for 2 weeks) and iron-sufficient groups. 1, 2, and 3 are those using 200, 500, and 700 bp PCR fragments, respectively.
Fig. 4 shows the results of measuring reductase activities of the invention by HPLC, in which the value of reductase activity is measured as the quantity of DMA by HPLC. 1, 2, 3, 4, 5, 6, and 7 show the cases of DMA alone, reductase gene1, reductase gene2, reductase gene5, reductase gene7, NAAT + NaBH₄, and vector control (PYH23), respectively.
Fig. 5 is photos in place of drawings showing the results of Northern blot analyses where the changes at elapsed times were examined for response of the reductase genes of the invention to iron-deficiency. The experiments were carried out using the barley roots on the 0, 2, 4, 7, 14th days of the iron-deficient treatment and on the 5th day of restart giving iron after the 14 days' iron-deficient treatment. 1 and 2 show the cases of the reductase genes 1 and 2(5), respectively.
Fig. 6 shows the cDNA base sequence and the deduced amino acid sequence of the reductase gene 1 of the invention.
Fig. 7 shows the cDNA base sequence and the deduced amino acid sequence of the reductase gene 2 of the invention.
Fig. 8 shows the cDNA base sequence and the deduced amino acid sequence of the reductase gene 5 of the invention.
Fig. 9 shows comparison of the amino acid sequences of the reductase gene 1, the reductase gene 2(5) of the invention, and glutathione reductase of the rice.
Fig. 10 is a photo in place of a drawing showing the response expressed against metal stress of the reductase gene of the invention and the glutathione reductase gene. In Fig. 10, DMAS and GR mean the reductase gene of the invention and the glutathione reductase gene, respectively. R and S mean the root and shoot (leaf, stem, etc.) portions, respectively.
Fig. 11 shows the amino acid sequences of the probe regions used when examining the response expressed against metal stress of the reductase gene of the invention and the glutathione reductase gene. In Fig. 11, DMAS1 and DMAS2 mean the reductase genes of the invention, and GR means the glutathione reductase gene.
Also in Fig. 11, the upper part enclosed with a slender box denotes the probe region. used when observing the response of GR to the metals, and the part from upper to lower enclosed with a wide box denotes the probe regions used when observing the response of DMAS to the metals.

### Best Mode for Carrying Out the Invention

The present invention relates to a reductase reducing keto-nicotianamine into 2'-deoxymugineic acid and a gene encoding the same.

Also the present invention relates to transformants and plants into which the gene encoding the reductase reducing keto-nicotianamine into 2'-deoxymugineic acid is transferred.

Further, the present invention relates to enzyme kits for biosynthesis of mugineic acids which are iron chelators which contain the reductase reducing the keto body of nicotianamine into 2'-deoxymugineic acid and at least one type of the other enzymes for biosynthesizing mugineic acids which are iron chelators, and to gene kits for constructing a plant having a enhanced tolerance to iron-deficiency which comprise genes encoding these enzymes.

Many of the enzymes and proteins involved in the biosynthetic pathways of mugineic acids have been successfully purified or the genes thereof have been successfully isolated. However, no enzyme which catalyzes the reaction from the keto body to DMA and no gene encoding the same have been successfully purified and isolated yet. Thus, the present inventors attempted to isolate a gene encoding an enzyme which catalyzes the reaction from the keto body to DMA in the biosynthetic pathways of mugineic acids. Although this gene has been reported to be an NAD(P)H-dependent enzyme by the uptake experiment by Shojima 10 years ago (Shojima et al., Plant Physiol., 93:1497-1503, 1990), thereafter, the study has not achieved the purification and isolation of the protein and gene thereof.

When deduced from the secretion amount of DMA, the amount of the endogenous reductase in rice may be lesser than that in barley, and thus the isolation from barley was attempted.

First, an enzyme families to which the target reductase might belong were searched, and as a result, the aldo-keto reductase gene superfamily (Seery et al., J. Mol. Evol., 46:139-146, 1998) appeared to be most proper, which widely covers NADH- and NADPH-dependent reductases. Then, the following two regions were chosen based on the amino acid sequences comparatively conserved in the family. 5'-YTIGAYTAYBTIGAC-3' (5'-side) 5'-RTGRCAYTCIAYYTG-3' (3'-side) The degenerate primers were designed based on the base sequences coding for the sequences of the two regions.

PCR where the template was a cDNA library from the barley (Hordeum vulgare L. cv. Ehimehadaka no.1) roots in an iron-deficient state was carried out using these primers.

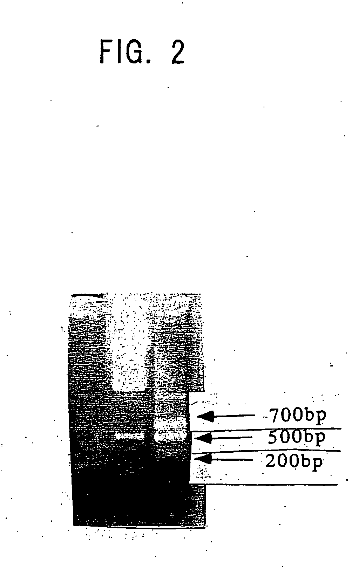

As a result, three PCR fragments (200, 500, and 700 bp) were obtained (Fig. 2). Fig. 2 is a photo in place of a drawing showing three PCR fragments obtained. The right side bands in Fig. 2 show 200, 500, and 700 bp fragments, respectively from the bottom.

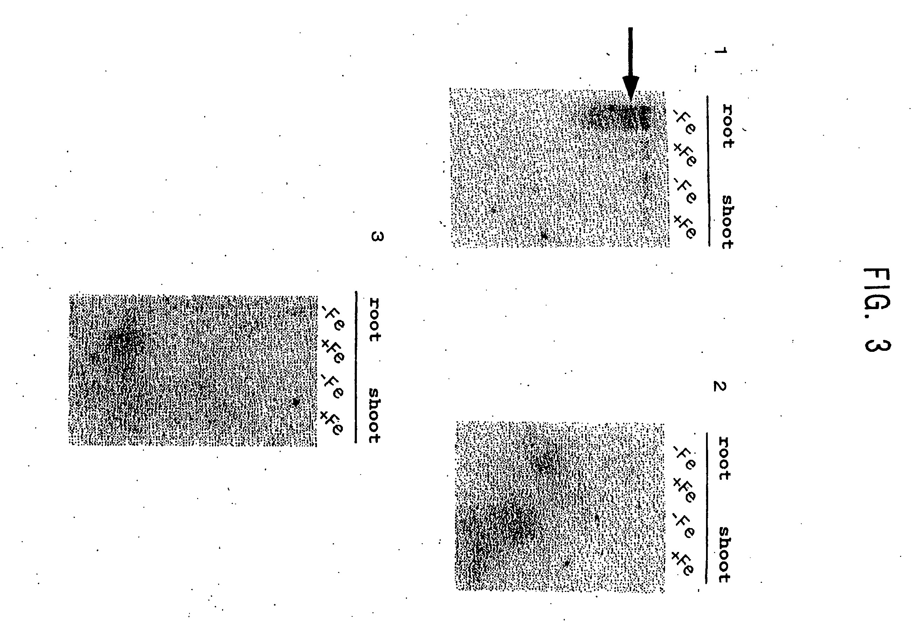

In order to examine that the obtained PCR fragments are the target gene, the gene expression was analyzed by Northern blotting in the root and shoot portions of the barley in the iron-deficient (-Fe) and sufficient (+Fe) groups. The results are shown in Fig. 3 by photos in place of drawings. The upper left photo 1 in Fig.3 is of the case using the 200 bp PCR fragment, the upper right photo 2 in Fig. 3 is of the case using the 500 bp PCR fragment, and the lower middle photo 3 is of the case using the 700 bp PCR fragment. In each photo, the left two lanes are from the root portion and the right two lanes are from the shoot portion. In each root and shoot portions, -Fe denotes the iron-deficient group (for two weeks) and +Fe denotes the iron-sufficient group.

For those where 500 and 700 bp PCR segments were used as probes, no gene expression was observed in both iron-deficient and sufficient groups. On the other hand, for those using 200 bp PCR segment, a band expressed in all conditions and a band expressed only in the root portion of the iron-deficient group were detected (see Fig. 3-1). All the genes of enzymes which catalyze the reactions before and after the pathway to produce DMA from the keto body in the biosynthetic pathways of mugineic acids are expressed only in the barley root with iron-deficiency. From this, it appears to be highly possible that the target gene is expressed in the barley root only with iron-deficient condition. Thus, it was believed that the 200 bp PCR fragment might contain the base sequence of the target gene, and cDNA clone containing this base sequence was isolated.

The isolation of cDNA clones was carried out by colony hybridization using one of PCR fragments which was detected in the iron-deficient condition by the Northern analysis as a probe. The iron-deficient barley root cDNA library was applied on eight LB plates containing ampicillin such that a clone density was from 25000 to 50000 clones/plate. The second and third screening was carried out such that a positive clone in the first screening is a single clone. As a result, four positive clones were obtained (named 1, 2, 5, and 7, respectively). The cDNA clones obtained here were sequenced. The base sequences of these genes all showed the highest homology to glutathione reductase of the rice (Kaminaka et al., Plant Cell Physiol., 39:1269-1280, 1998).

Next, for these cDNA clones, enzymatic activities were examined using the proteins obtained by transforming yeast with these cDNA. Since the keto body which is a substrate is an unstable compound, nicotianamine which is a precursor thereof was used as the starting material. The assay was carried out according to NAAT activity measurement method by Takahashi's method (Takahashi, et al., Plant Physiol., 121:947-956, 1999). That is, nicotianamine was reacted to the keto body in the presence of NAAT, and subsequently the protein expressed by the yeast transformant was used in place of chemical reduction by NaBH₄. The protein expressed by an empty vector (pYH23) without the insert was used as a control. The value of activity was measured by detecting the quantity of DMA using HPLC.

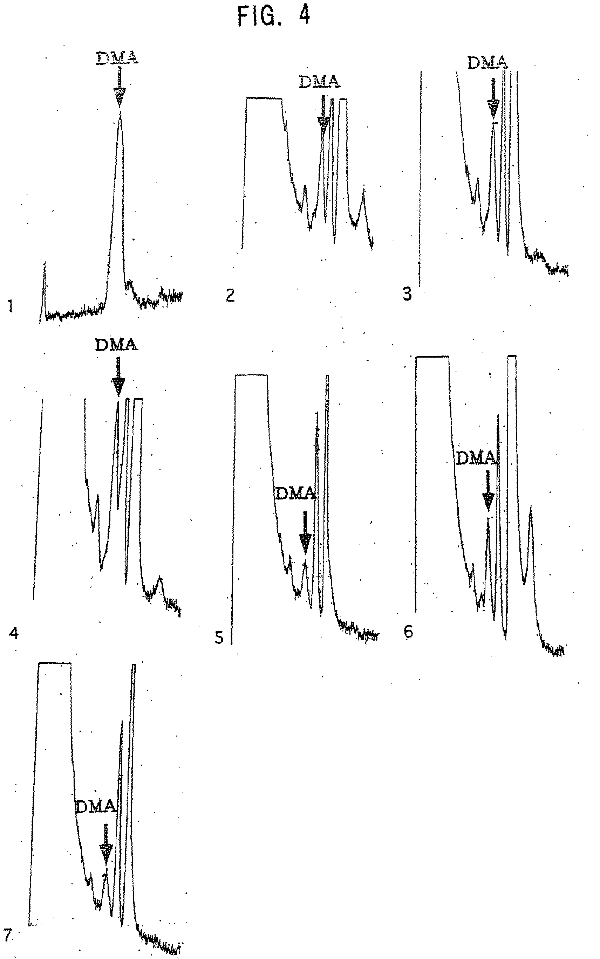

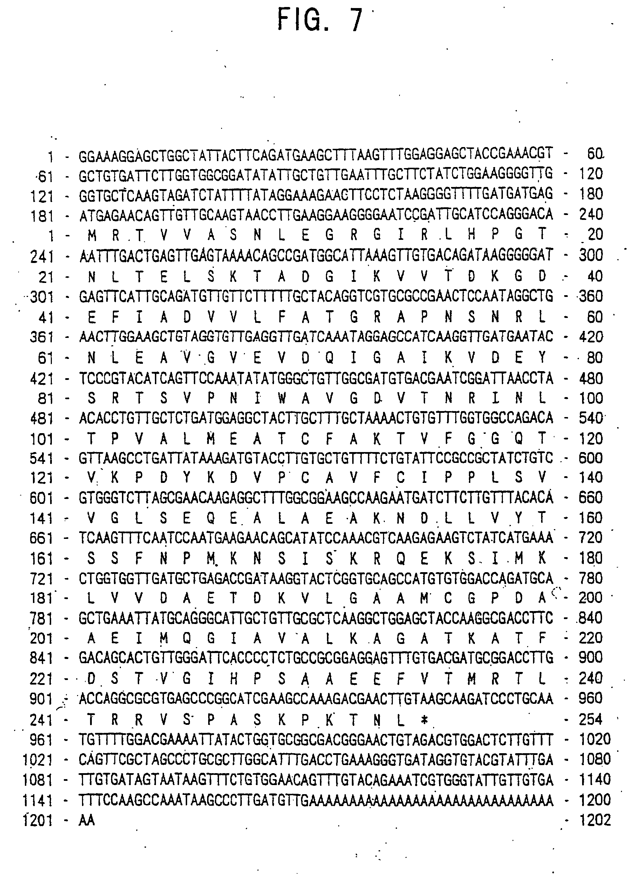

The results of HPLC were shown in Fig. 4. Fig. 4-1 is of the case of DMA alone and shows a retention time in the standard DMA. Fig. 4-2 is of the case where the reductase gene 1 was expressed. Fig. 4-3 is of the case where the reductase gene 2 was expressed. Fig. 4-4 is of the case where the reductase gene 5 was expressed. Fig. 4-5 is of the case where the reductase gene 7 was expressed. Fig. 4-6 is of the case of chemical reduction by NaBH₄. Fig. 4-7 is of the case where the control vector (PYH23) was expressed.

Those were determined to be positive in which the more amount of DMA was observed relative to that of the case of the vector alone (Fig. 4-7). As a result, DMA was detected in all proteins obtained from the cDNA except the reductase gene 7 (Fig. 4-5). Among them, the highest activity was observed in the proteins from the cDNA of the reductase genes 2 (Fig. 4-3) and 5 (Fig. 4-4).

Next, the response to iron-deficiency was observed in the cases of 1, 2 and 5 where the DMA activity was detected.

Among the resultant cDNA clones having the DMA activity, the expression of the reductase genes 1, 2 and 5 was analyzed for the elapsed change of the response to iron-deficiency by Northern blotting. Total six samples of RNA were used, i.e., the barley root just before getting iron-deficiency (0 day), those on the 2nd, 4th, 7th and 14th day after getting iron-deficiency, and one to which iron was given for 5 days after 14 days' iron-deficiency. Electrophoresis and imaging of the RNA were carried out as is the case with the Northern analysis using PCR fragments.

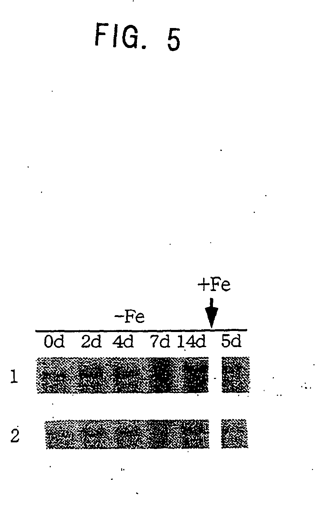

The results are shown in the photos in place of drawings in Fig. 5. Fig. 5-1 is of the case using the reductase gene 1, and Fig, 5-2 is of the case using the reductase gene 2. The notes, 0d, 2d, 4d, 7d, 14d and 5d in Fig. 5 denotes the barley root of 0, 2nd, 4th, 7th, 14th days after the treatment of iron-deficiency and 5th day after the re-administration of iron following 14 days' iron-deficient treatment, respectively.

As a result, it was found that the expression of all genes was induced with the progress of iron-deficiency and further that the expression was reduced by giving iron (Fig. 5-5d).

Next, the cDNA clones of the reductase genes 1, 2 and 5 having the DMA activity were sequenced. As a result, the reductase genes 2 and 5 shared the identical base sequence for the open reading flame (ORF) portion. The ORF of the reductase gene 1 has additional 315 bp at the 5' end of the other two ORF.

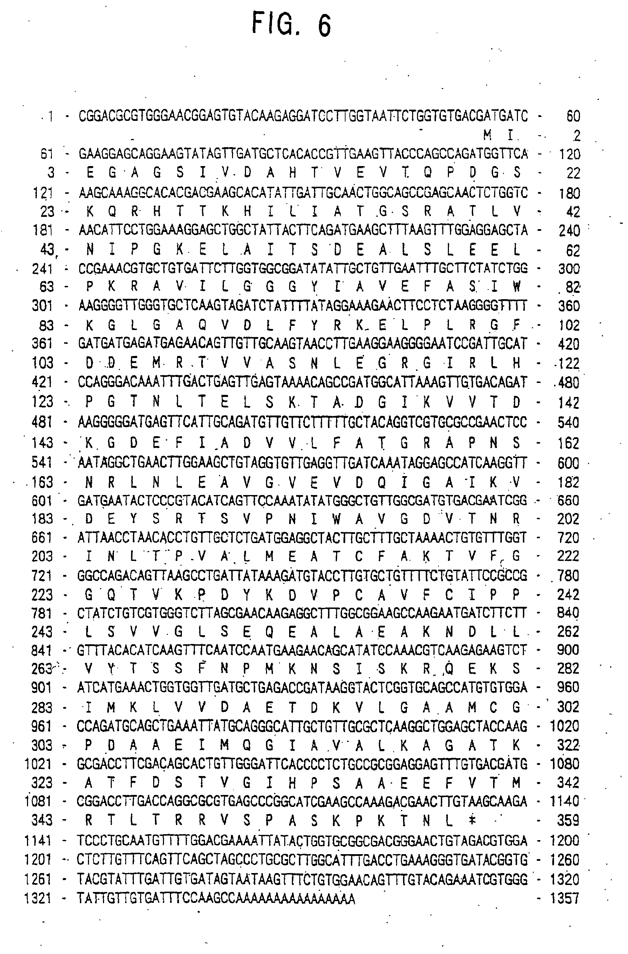

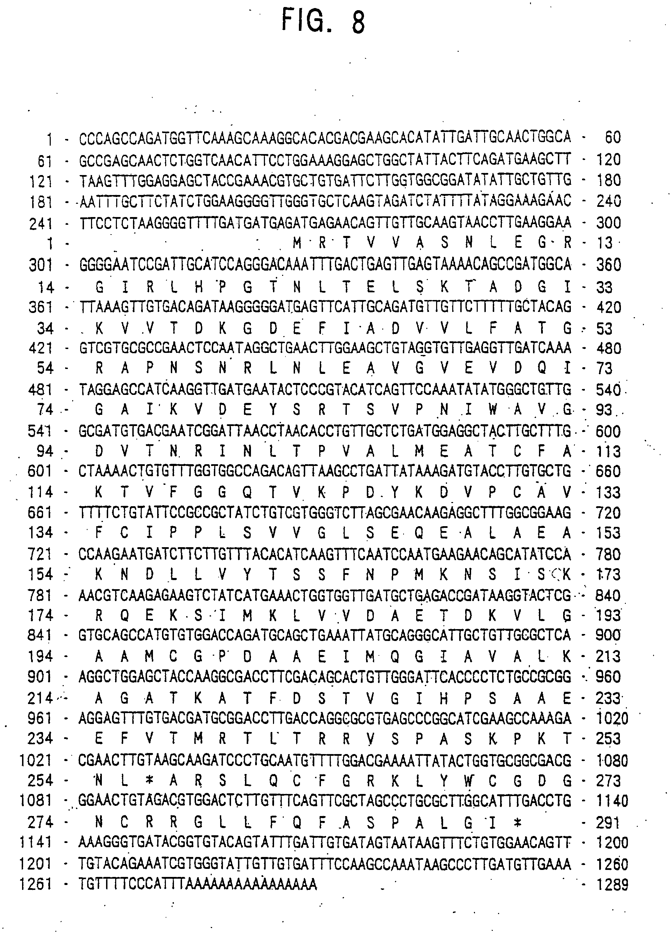

The amino acid sequences of the reductase genes 1 and 2 are shown in the sequence numbers 1 and 2, respectively in the sequence table. Also, the base sequences of the reductase genes 1, 2 and 5 are shown in the sequence numbers 3, 4 and 5, respectively in the sequence table. Furthermore, the base sequences and the deduced amino acid sequences of the reductase genes 1, 2 and 5 are shown in Figs. 6, 7 and 8, respectively.

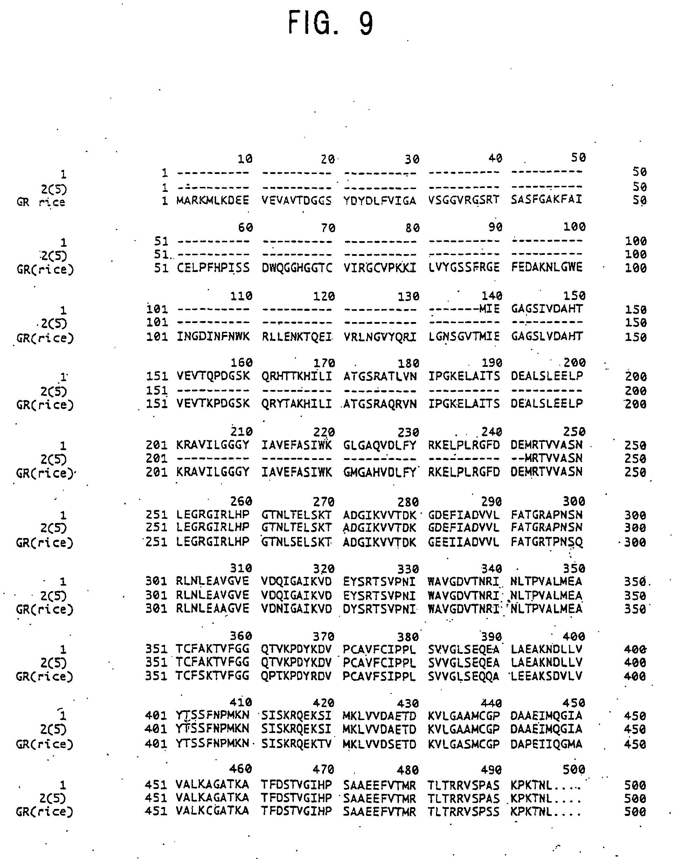

Next, information obtained from the deduced amino acid sequence based on the determined base sequence was examined. Each amino acid sequence has 89.5% homology to glutathione reductase in the rice (see Fig. 9). In Fig. 9, the upper line shows the amino acid sequence of the reductase gene 1, the middle line shows the amino acid sequence of the reductase gene 2 or 5, and the bottom line shows the amino acid sequence of glutathione reductase in the rice.

Next, the localization of the obtained proteins was predicted by PSORT. As a result, it was found that the protein from the reductase gene 1 might localize in plasma membranes, and that the protein from the reductase gene 2 (5) might localize in endoplasmic reticulums (membranes). On the other hand, glutathione reductase in the rice which exhibited the highest homology of 89.3% to the reductase genes of the present invention in the amino acid sequence showed the highest probability, of its cytoplasmic localization.

The reductases and their genes of the present invention exhibited no homology to the members of NAD(P)H dependent reductase family, based on which the degenerate primers were designed, and exhibited the highest homology to glutathione reductase. The localized site of the gene 2 (or 5) predicted by its amino acid.sequence was the membrane of endoplasmic reticulum. This and the fact that NAS and NAAT involved in the biosynthetic pathways of mugineic acids are localized in the granules derived from the rough surfaced endoplasmic reticulum specific for the barley root with iron-deficiency lead to a high possibility that the isolated gene 2 or 5 may be the target gene. Moreover, the expression of these genes was induced by the iron-deficiency treatment and reduced by giving iron in the time-elapsed change of response to iron-deficiency, showing that these genes have responsiveness to iron.

Taken together, the genes of the present invention are the reductases which catalyze the reaction from the keto body to DMA in the biosynthetic pathways of mugineic acids, and it can be said that a novel rice species having an enhanced tolerance to iron-deficiency is potentially developed in the future.

Thus, the reductase of the present invention is the reductase which reduces keto-nicotianamine into 2'-deoxymugineic acid, and the present invention has elucidated the entire enzyme group for biosynthesizing mugineic acids which are natural iron-chelators collectively referred to as phytosiderophores in the manner of iron acquisition mechanism of the plant referred to as "Strategy II".

Therefore, the plant can get the iron acquisition mechanism referred to as "Strategy II" and its tolerance to iron-deficiency is enhanced by introducing these enzymes or the genes encoding these enzymes into the plant.

For NAAT which is one type in the enzyme group for biosynthesizing mugineic acids, the transformed rice has been produced by introducing naat genome. NAAT is the enzyme that catalyzes the reaction from NA to the keto body in the biosynthetic pathway of mugineic acids, and it has been found that the plant can grow in the iron-deficient soil without getting chlorosis by introducing this gene. It has been thought that in the rice transformed with the NAAT gene alone, the expression induction of the naat gene due to iron-deficiency produces excess amounts of the keto body and the excess keto body is converted to DMA by the endogenous reductase, resulting in growing in the iron-deficient soil without getting chlorosis.

However, the quantity of the endogenous reductase in rice estimated from the secretion quantity of DMA is lesser than that in barley, and it is highly possible that the excessively produced keto body by the expression induction of naat gene is not efficiently converted to DMA. Based on this idea, it is anticipated that the rice transformed with the gene of the present invention encoding the enzyme which catalyzes the reaction from the keto body to DMA together with the naat genome may convert the excessively produced keto body by NAAT to DMA efficiently by the reductase and may become far stronger to iron-deficiency relative to the rice transformed with the naat genome alone. In the light of this point, it can be said that it is quite important that the reductase which catalyzes the reaction from the keto body to DMA is isolated.

The present invention provides the reductase which reduces keto-nicotianamine into 2'-deoxymugineic acid in the course of the biosynthesis of mugineic acids participating in the iron acquisition mechanism in plants. The reductase of the present invention is preferably one having the amino acid sequence described in the sequence number 1 or 2 in the sequence table. In this amino acid sequence, one or more amino acids may be substituted with the other amino acids, one or more amino acids may be deleted, and one or more amino acids may be further added as long as the protein has the activity reducing keto-nicotianamine into 2'-deoxymugineic acid in the course of the biosynthesis of mugineic acids. Also, those may have the amino acid sequence taking place in the combination of these substitution, deletion and addition.

Also, the present invention provides the gene encoding the aforementioned reductase of the invention. The preferable base sequence of the gene of the present invention is made up of, but is not limited to the base sequence described in either the sequence number 3, 4 or 5 in the sequence table. The base sequence can be modified into an easily expressed sequence as long as it encodes the aforementioned amino acid sequence. Also, the genes of the present invention include the genes made up of sequences complementary to these base sequences and base sequences capable of hybridizing to the base sequence under the stringent condition.

Additionally, the base sequences made up of the partial genes of the invention are also included in the invention. These partial sequences can be also used as primers for PCR, and the present invention includes partial sequences used for such methods.

Also, the gene of the present invention may have a promoter region required at upstream thereof. Such promoters are not limited to the following sequence table, and the promoter suitable for the plant can be chosen depending on the plant into which the gene of the invention is transferred.

The present invention provides transformants transformed with the aforementioned gene of the invention. Various cells such as bacteria such as Escherichia coli, animal cells such as hamster cells, plant cells and yeasts can be employed as host cells for the transformants of the invention. The reductase of the present invention can be expressed and produced by these transformants.

Also, the present invention provides the plant into which the aforementioned gene of the invention is introduced. Since the full-picture of the enzyme group involved in the biosynthesis of mugineic acids has been demonstrated by elucidating the reductase of the present invention, it becomes possible that a set of the genes encoding these enzyme group is transferred into the plants which do not have Strategy II where mugineic acids acts iron chelators as the iron acquisition mechanism to newly impart the iron acquisition mechanism by Strategy II. Therefore, the plants into which the gene of the present invention is introduced are not limited to Graminaceous plants having the iron acquisition mechanism by strategy II, but the introduction into Graminaceous plants is preferred.

Although enhanced tolerance to iron-deficiency can be also anticipated even by introducing the gene of the invention alone, the plants having more enhanced tolerance to iron-deficiency can be obtained by preferably introducing the gene encoding the other enzyme, preferably naat simultaneously.

As mentioned above, all enzymes in the biosynthetic pathways of mugineic acids shown in Fig. 1 have been elucidated by the elucidation of the reductase of the present invention, and their full-picture was demonstrated. Therefore, the present invention provides the enzyme group for the biosynthesis of mugineic acids involved in Strategy II. That is, the present invention provides the enzyme kits for biosynthesizing mugineic acids which are iron chelators made up of "S-adenosylmethionine synthase" which converts methionine into S-adenosylmethionine (SAM), "nicotianamine synthase" which converts it into nicotianamine (NA), "nicotianamine amino group. transferase (NAAT)" which converts it into the keto body, and "reductase" of the invention, and further "IDS3" which further converts it into mugineic acid, and the like. The enzyme kits of the present invention makes the reductase.of the invention as an essential enzyme and contains at least one of the other enzymes, and preferably are the kits containing all enzymes capable of completing the biosynthesis from methionine. Using the enzyme kit of the present invention, mugineic acids can be readily produced from methionine or products of their biosynthetic course.

The present invention provides the gene kits containing the genes encoding respective enzymes of the aforementioned enzyme group in order to construct the plants having enhanced tolerance to iron-deficiency. The plants having enhanced tolerance to iron-deficiency can be constructed by introducing these genes into the plants. The plants having enhanced biosynthetic process from methionine to mugineic acids can be constructed by introducing the genes encoding respective enzymes for all aforementioned enzymes.

The gene of each enzyme to be introduced into a plant is preferably a gene containing the promoter region of the plant into which the gene is introduced. Also, the plants into which each gene is introduced are not limited to, but are preferably Graminaceous plants.

### Examples

Next, the present invention is described in more detail by examples, but is not limited to these examples.

### Example 1. Cultivation of barley

The seeds of barley (Hordeum vulgare L. cv. Ehimehadaka no.1) were placed on paper towel wetted with distilled water by shielding light with aluminium foil at room temperature for 3 days to induce germination. The seedlings were transferred onto a net floated on a hydroponic solution and grown with aerating by a pump. When the second leaf was emerged, the plants were transferred to a 20 L container and the hydroponic growth was continued.

The composition of the hydroponic solution was [7×10⁻⁴ M K₂SO₄, 1×10⁻⁴ M KCl, 1×10⁻⁴ M KH₂PO₄, 2×10⁻³ M Ca(NO₃)₂, 5×10⁻⁴ M MgSO₄, 1×10⁻⁵ M H₂BO₃, 5×10⁻⁷ M MnSO₄, 5×10⁻⁷ M ZnSO₄, 2×10⁻⁷ M CuSO₄, 1×10⁻⁸ M (NH₄)₆MoO₂₄, 1.5×10⁻⁴ M Fe-EDTA].

The pH value of the hydroponic solution was adjusted with 1N HCl everyday so as to be around pH 5.5. The hydroponic solution was prepared using distilled water and replaced every week. When the fourth leaf was emerged, the roots were washed with distilled water to remove adhered iron, subsequently transferred to the hydroponic solution without iron to start the treatment of iron-deficiency, and cultivated until the target time period.

### Example 2. Design of degenerate primers and PCR using them

First, the experiment was started by searching enzyme families to which the target reductase seems to belong. As a result, it was thought that aldo-keto reductase gene super family which widely covers NADH-, NADPH-dependent reductases was the most suitable. Next, two regions were chosen based on the amino acid sequence relatively conserved in the family. The degenerate primers were designed based on the base sequences replaced for the sequences of the two regions. The degenerate primers were synthesized.

LDYV/LD....5'-YTIGAYTAYBTIGAC-3' (5'-end)

QV/IECH....5'-RTGRCAYTCIAYYTG-3' (3'-end)

Using these primers, PCR was carried out using the cDNA library of Fe-deficient barley root as a template.

As a result, three fragments, i.e., 200, 500 and 700 bp PCR fragments were obtained. The results are shown in Fig. 2.

### Example 3. Selection of PCR fragments induced in Fe-deficient barley roots

The objectives appear to be induced in Fe-deficient roots among the fragments amplified by PCR, and thus, Northern analysis was used as the method for selection thereof. RNA was extracted from those sampled from the root and shoot portions of the barley with the treatment of 2 weeks' Fe-deficiency. The method for extracting RNA was as follows.

The plants crushed using liquid nitrogen were dissolved in an extraction buffer (150 mM LiCl, 5 mM EDTA, pH8, 5% SDS, 80 mM Tris-HCl, pH9). Next, phenol-chloroform extraction was carried out twice. To its supernatant, 0.3 M sodium acetate and one part volume of isopropanol were added and centrifuged. The pellet was dissolved in 3 M lithium chloride and ice-cooled for 10 min followed by centrifugation. The pellet was washed with 80% ethanol, and dissolved in sterile water. The storage was carried out at -80°C. The resultant RNA was electrophoresed on 1.2% agarose gel, and subsequently blotted onto a membrane (Hybond-N+, Amersham-Pharmacia).

The probes used for hybridization were made by random primer DNA labeling kit ver. 2.0 (Takara) using the PCR fragments. The hybridization was carried out at 65°C for 18 hours using Church hybridization solution (0.5 M Church phosphate buffer, 1 mM EDTA, 7% (w/v) SDS). The membrane was washed twice with Church hybridization washing solution (40 mM Church phosphate buffer, 1% (w/v) SDS) at 65°C for 10 min. After washing, the membrane was wrapped with Saran Wrap and exposed to IP imaging plate (Fuji Film) in a cassette overnight. The image processing was carried out with BAS2000 image analyzer (Fuji Film).

The results are shown in Fig. 3. Fig. 3-1, 3-2 and 3-3 are of the cases using the PCR fragments of 200, 500 and 700 bp, respectively.

### Example 4. Screening

The isolation of the cDNA clones was carried out by colony hybridization using those induced in Fe-deficient roots detected by Northern analysis as probes. The Fe-deficient barley root cDNA library was applied onto 8 LB plates containing ampicillin such that a clone density was from 25000 to 50000 clones/plate. The second and third screening were carried out such that the positive clone in the first screening becomes a single clone. The finally resultant cDNA clones were sequenced.

### Example 5. Selection by activity measurement

The enzymatic activity was examined for the cDNA clones obtained from the screenings. Those obtained by transforming yeast with the cDNA were used as the proteins used. Since the keto body in the biosynthetic pathways of mugineic acids is an unstable compound, nicotianamine which is a precusor thereof was used as a starting material. The assay was carried out according to NAAT activity assay method by Takahashi's method (Takahashi, et al., Plant Physiol., 121:947-956, 1999). Nicotianamine was reacted by NAAT to produce the keto body, and subsequently, the protein expressed from the resultant cDNA clones was reacted in place of the chemical reduction with NaBH₄. The proteins used were obtained by crushing the yeast and trapping the proteins with molecular weight of 30 kDa or more with ultrafree C3-LTK (Japan Millipore Co.). The enzymatic reaction was carried out on the trapping cup. The buffer used for the reaction of the reductase was 0.1 M Tris, 0.1 mM EDTA, 10 mM NAD(P)H, pH8.5. The protein expressed from the vector (pYH23) without the insert was used as a control. The value of the activity was determined by detecting the quantity of DMA using HPLC.

The results were shown in Fig. 4. Fig. 4-1 is of the case of DMA alone and shows a retention time in the standard DMA. Fig. 4-2 is of the case where the reductase gene 1 was expressed. Fig.4-3 is of the case where the reductase gene 2 was expressed. Fig.4-4 is of the case where the reductase gene 5 was expressed. Fig.4-5 is of the case where the reductase gene 7 was expressed. Fig. 4-6 is of the case of chemical reduction by NaBH₄. Fig. 4-7 is of the case where the control vector (PYH23) was expressed.

Those were determined to be positive in which the more amount of DMA was observed relative to that of the case of the vector alone (Fig. 4-7). As a result, DMA was detected in all proteins obtained from cDNA except the reductase gene 7 (Fig. 4-5). Among them, the highest activity was observed in the proteins from cDNA of the reductase genes 2 (Fig. 4-3) and 5 (Fig. 4-4).

### Example 6. Northern analysis

Among the resultant cDNA clones, those having the DMA activity were analyzed for the elapsed change of the response to Fe-deficiency by Northern blotting. Total six samples of RNA were used, i.e., the barley root just before getting Fe-deficiency (0 day), those on the 2nd, 4th, 7th and 14th day after getting Fe-deficiency, and one which iron was given for 5 days after 14 days' Fe-deficiency. Electrophoresis and imaging of the RNA were carried out as is the case with the northern analysis of the PCR fragments.

The results are shown in Fig. 5. Fig. 5-1 is of the case using the reductase gene 1, and Fig, 5-2 is of the case using the reductase gene 2. The notes, 0d, 2d, 4d, 7d, 14d and 5d in Fig. 5 denotes the barley roots of 0, 2nd, 4th, 7th, 14th days after the treatment of iron-deficiency and 5th day after the re-administration of iron following 14 days' iron-deficient treatment, respectively.

As a result, it was found that the expression of all genes was induced with the progress of iron-deficiency and further that the expression was reduced by giving iron (Fig. 5-5d).

### Example 7. Expression response of the reductase gene of the invention and glutathione reductase gene to metallic stress

When compared the base sequence of the reductase genes (DMAS1 and DMAS2) in the rice with that of the glutathione reductase gene (GR) in the rice, it is shown that the formers are the parts of GR. Therefore, there is a possibility that GR per se was induced by Fe-deficiency and the glutathione reductase per se which is a product thereof had DMAS activity. In order to investigate this possibility, the gene expression was examined using the base sequence belonging to only 5' upstream of GR gene as the probe under the metallic stress of iron and zinc by the Northern method.

### (1) Plant materials

The seeds of barley (Hordeum vulgare L. cv. Ehimehadaka no. 1) were placed on paper towel wetted with distilled water by shielding light with aluminium foil at room temperature for 3 days to induce germination. The seedlings were transferred onto a net floated on a hydroponic solution and grown with aerating by a pump. When the second leaf was emerged, the plants were transferred to a 20 L container and the hydroponic growth was continued. The composition of the hydroponic solution was [7×10⁻⁴ M K₂SO₄, 1×10⁻⁴ M KCl, 1×10⁻⁴ M KH₂PO₄, 2×10⁻³ M Ca(NO₃), 2.5×10⁻⁴ M MgSO₄, 1×10⁻⁵ M H₂BO₃, 5×10⁻⁷ M MnSO₄, 5×10⁻⁷ M ZnSO₄, 2×10⁻⁷ M CuSO₄, 1×10⁻⁸ M (NH₄)₆Mo₇O₂₄, 1.5×10⁻⁴ M Fe-EDTA]. The pH value of the hydroponic solution was adjusted with 1N HCl everyday so as to be around pH 5.5. The hydroponic solution was prepared using distilled water and replaced every week. When the fourth leaf was emerged, the roots were washed with distilled water to remove adhered metals, subsequently transferred to the hydroponic solution without each metal (Fe, Zn) to start the treatment of metal-deficiency (Fe, Zn), and cultivated until the target time period.

### (2) Northern analysis

The root and shoot portions of the barley with metal deficiency (Fe,Zn) were sampled and RNA was extracted.

The method for extracting RNA is as follows.

The plants frozen in liquid nitrogen were ground in a mortar and were dissolved in an extraction buffer (150 mM LiCl, 5 mM EDTA, pH8. 5% SDS, 80 mM Tris-HCl, pH9). Next, phenol-chloroform extraction was carried out twice. To its supernatant, 0.3 M sodium acetate and one part volume of isopropanol were added and centrifuged. The pellet was dissolved in 3 M lithium chloride and ice-cooled for 10 min followed by centrifugation. The pellet was washed with 80% ethanol, and dissolved in sterile water. The storage was carried out at -80°C.

The resultant RNA was electrophoresed on 1.2% agarose gel, and subsequently blotted onto a membrane (Hybond-N+, Amersham-Pharmacia).

The probes used for hybridization were made by labeling the PCR fragments with random primer DNA labeling kit ver. 2.0 (Takara). The hybridization was carried out at 65°C for 18 hours using Church hybridization solution (0.5 M Church phosphate buffer, 1 mM EDTA, 7% (w/v) SDS). The washing was carried out twice with Church hybridization washing solution (40 mM Church phosphate buffer, 1.% (w/v) SDS) at 65°C for 10 min. After washing, the membrane was wrapped with Saran Wrap and exposed to IP imaging plate (Fuji Film) in a cassette overnight. The image processing was carried out with BAS3000 image analyzer (Fuji Film).

### (3) Results and discussion

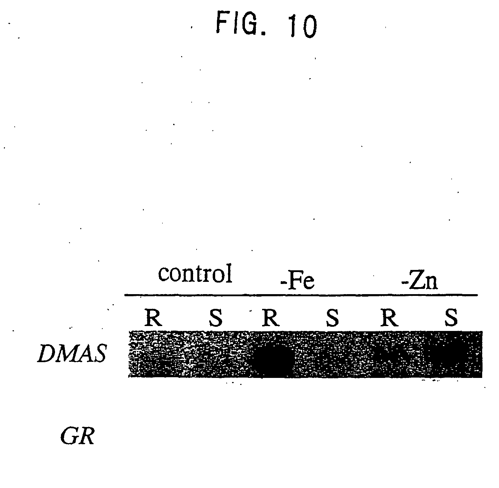

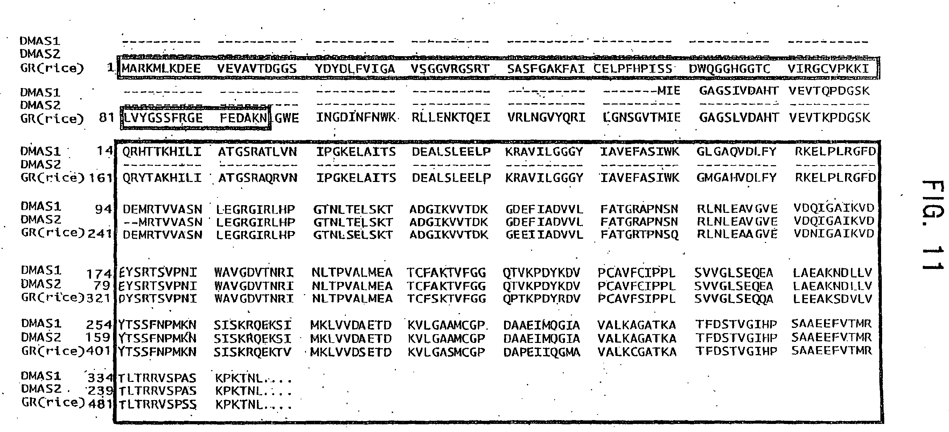

The result of Northern analysis using the sequence of DMAS1 (corresponding to the common sequence of DMAS1, DMAS2 and GR which is widely enclosed with a box in Fig. 11) as the probe is shown in Fig. 10. This is strongly expressed in the root portion with Fe-deficiency (additionally, it is relatively strongly expressed in the root and shoot (leaf, stem, etc.) portions with Zn-deficiency). However, no expression was observed in the control group and under any of stresses in the case using the GR-inherent sequence (sequence enclosed in a slender box in Fig. 11) as the probe. These two results mean that GR gene expression is not related to Fe-deficiency, and indicate that it is the gene which is scarcely expressed in a usual condition. Conversely, the results indicates that DMAS1 and DMAS2 are the genes which are strongly expressed in the root portion of the barley with Fe-deficiency. Therefore, it can be concluded that DMASs are the enzymes of which gene expression is induced under Fe-deficient condition and which synthesize deoxymugineic acid from the keto body. Although genomic gene of DMAS has not been cloned yet, it is thought that still unknown response system elements to Fe-deficiency might be included in 5' upstream of this genomic gene.

### Industrial Availability

The present invention provides the reductase which reduces keto-nicotianamine into 2'-deoxymugineic acid in the course of the biosynthesis of mugineic acids participating in the iron acquisition mechanism of plants, the gene encoding the same and plants having enhanced tolerance to iron-deficiency owing to the above gene transferred thereinto. Enhanced tolerance to iron-deficiency of the plants enables plant cultivation even in the soil where. supply of iron is difficult such as alkali soils, expands cultivated acreage capable of cultivation, and enables to resolve food and environmental problems in the future. Also, the full-picture of the biosynthetic pathways of mugineic acids in the plant could be elucidated by the elucidation of the reductase of the present invention. Therefore, it became possible to construct the plants having highly enhanced tolerance to iron-deficiency using the enzyme group involved in these biosyntheses and the genes encoding the same.

Therefore, the present invention provides a series of enzyme system required for the biosynthesis of mugineic acids and a series of gene system encoding the same, and also enables to impart the iron acquisition mechanism by Strategy II to the plants.

## Claims

1. A reductase reducing keto-nicotianamine into 2'-deoxymugineic acid.

2. The reductase according to claim 1, wherein the reductase is derived from barley.

3. The reductase according to claim 1 or 2, comprising the amino acid sequence described in the sequence number 1 or 2 in the sequence table or an amino acid sequence comprising deletion, addition or substitution of one or more amino acids in the amino acid sequence.

4. A gene encoding the reductase according to any of claims 1 to 3.

5. The gene according to claim 4, wherein the gene comprises the base sequence described in the sequence number 3, 4 or 5 in the sequence table or a base sequence capable of hybridizing to the base sequence under a stringent condition.

6. A transformant transformed with the gene according to claim 4 or 5.

7. The transformant according to claim 6, wherein a host cell of the transformant is a plant cell.

8. A plant into which the gene according to claim 4 or 5 is transferred.

9. The plant according to claim 8, wherein the plant is Graminaceous plant.

10. The plant according, to claim 8 or 9 which is the plant having a enhanced tolerance to iron-deficiency.

11. An enzyme kit for biosynthesizing mugineic acids which are iron chelators, which contains the reductase according to claims 1 to 3 and at least one of the other enzymes for biosynthesizing mugineic acids which are iron chelators.

12. A gene kit for constructing a plant having an enhanced tolerance to iron-deficiency which contains the gene according to claim 4 or 5 and a gene encoding at least one of the other enzymes for biosynthesizing mugineic acids which are iron chelators.

13. The gene kit according to claim 12, wherein the gene is a gene containing a promoter region of the plant into which the gene is transferred.

14. The gene kit according to claim 13, wherein the plant into which the gene is transferred is Graminaceous plant.


SEQUENCE LISTING

<110> Japan Science and Technology Corporation

<120> A deoxymugineic acid synthetase and its gene.

<130> N.88545 TAC

<140> 02702724.2
<141> 2002-03-04

<150> PCT/JP02/01940
<151> 2002-03-04

<150> JP2001-086162
<151> 2001-03-23

<160> 5

<210> 1
<211> 359
<212> PRT
<213> Hordeum vulgare L. cv. Ehimehadaka no.1

<400> 1
Met Ile Glu Gly Ala Gly Ser Ile Val Asp Ala His Thr Val Glu Val
1               5                   10                  15

Thr Gln Pro Asp Gly Ser Lys Gln Arg His Thr Thr Lys His Ile Leu
            20                  25                  30

Ile Ala Thr Gly Ser Arg Ala Thr Leu Val Asn Ile Pro Gly Lys Glu
        35                  40                  45

Leu Ala Ile Thr Ser Asp Glu Ala Leu Ser Leu Glu Glu Leu Pro Lys
    50                  55                  60

Arg Ala Val Ile Leu Gly Gly Gly Tyr Ile Ala Val Glu Phe Ala Ser
65                  70                  75                  80

Ile Trp Lys Gly Leu Gly Ala Gln Val Asp Leu Phe Tyr Arg Lys Glu
                85                  90                  95

Leu Pro Leu Arg Gly Phe Asp Asp Glu Met Arg Thr Val Val Ala Ser
            100                 105                 110

Asn Leu Glu Gly Arg Gly Ile Arg Leu His Pro Gly Thr Asn Leu Thr
        115                 120                 125

Glu Leu Ser Lys Thr Ala Asp Gly Ile Lys Val Val Thr Asp Lys Gly
    130                 135                 140

Asp Glu Phe Ile Ala Asp Val Val Leu Phe Ala Thr Gly Arg Ala Pro
145                 150                 155                 160

Asn Ser Asn Arg Leu Asn Leu Glu Ala Val Gly Val Glu Val Asp Gln
                165                 170                 175

Ile Gly Ala Ile Lys Val Asp Glu Tyr Ser Arg Thr Ser Val Pro Asn
            180                 185                 190

Ile Trp Ala Val Gly Asp Val Thr Asn Arg Ile Asn Leu Thr Pro Val
        195                 200                 205

Ala Leu Met Glu Ala Thr Cys Phe Ala Lys Thr Val Phe Gly Gly Gln
    210                 215                 220

Thr Val Lys Pro Asp Tyr Lys Asp Val Pro Cys Ala Val Phe Cys Ile
225                 230                 235                 240

Pro Pro Leu Ser Val Val Gly Leu Ser Glu Gln Glu Ala Leu Ala Glu
                245                 250                 255

Ala Lys Asn Asp Leu Leu Val Tyr Thr Ser Ser Phe Asn Pro Met Lys
            260                 265                 270

Asn Ser Ile Ser Lys Arg Gln Glu Lys Ser Ile Met Lys Leu Val Val
        275                 280                 285

Asp Ala Glu Thr Asp Lys Val Leu Gly Ala Ala Met Cys Gly Pro Asp
    290                 295                 300

Ala Ala Glu Ile Met Gln Gly Ile Ala Val Ala Leu Lys Ala Gly Ala
305                 310                 315                 320

Thr Lys Ala Thr Phe Asp Ser Thr Val Gly Ile His Pro Ser Ala Ala
                325                 330                 335

Glu Glu Phe Val Thr Met Arg Thr Leu Thr Arg Arg Val Ser Pro Ala
            340                 345                 350

Ser Lys Pro Lys Thr Asn Leu
        355

<210> 2
<211> 254
<212> PRT
<213> Hordeum vulgare L. cv. Ehimehadaka no.1

<400> 2
Met Arg Thr Val Val Ala Ser Asn Leu Glu Gly Arg Gly Ile Arg Leu
1               5                   10                  15

His Pro Gly Thr Asn Leu Thr Glu Leu Ser Lys Thr Ala Asp Gly Ile
            20                  25                  30

Lys Val Val Thr Asp Lys Gly Asp Glu Phe Ile Ala Asp Val Val Leu
        35                  40                  45

Phe Ala Thr Gly Arg Ala Pro Asn Ser Asn Arg Leu Asn Leu Glu Ala
    50                  55                  60

Val Gly Val Glu Val Asp Gln Ile Gly Ala Ile Lys Val Asp Glu Tyr
65                  70                  75                  80

Ser Arg Thr Ser Val Pro Asn Ile Trp Ala Val Gly Asp Val Thr Asn
                85                  90                  95

Arg Ile Asn Leu Thr Pro Val Ala Leu Met Glu Ala Thr Cys Phe Ala
            100                 105                 110

Lys Thr Val Phe Gly Gly Gln Thr Val Lys Pro Asp Tyr Lys Asp Val
        115                 120                 125

Pro Cys Ala Val Phe Cys Ile Pro Pro Leu Ser Val Val Gly Leu Ser
    130                 135                 140

Glu Gln Glu Ala Leu Ala Glu Ala Lys Asn Asp Leu Leu Val Tyr Thr
145                 150                 155                 160

Ser Ser Phe Asn Pro Met Lys Asn Ser Ile Ser Lys Arg Gln Glu Lys
                165                 170                 175

Ser Ile Met Lys Leu Val Val Asp Ala Glu Thr Asp Lys Val Leu Gly
            180                 185                 190

Ala Ala Met Cys Gly Pro Asp Ala Ala Glu Ile Met Gln Gly Ile Ala
        195                 200                 205

Val Ala Leu Lys Ala Gly Ala Thr Lys Ala Thr Phe Asp Ser Thr Val
    210                 215                 220

Gly Ile His Pro Ser Ala Ala Glu Glu Phe Val Thr Met Arg Thr Leu
225                 230                 235                 240

Thr Arg Arg Val Ser Pro Ala Ser Lys Pro Lys Thr Asn Leu
                245                 250

<210> 3
<211> 1357
<212> DNA
<213> Hordeum vulgare L. cv. Ehimehadaka no.1

<400> 3
cggacgcgtg ggaacggagt gtacaagagg atccttggta attctggtgt gacg atg      57
                                                            Met
                                                            1

atc gaa gga gca gga agt ata gtt gat gct cac acc gtt gaa gtt acc      105
Ile Glu Gly Ala Gly Ser Ile Val Asp Ala His Thr Val Glu Val Thr
            5                   10                  15

cag cca gat ggt tca aag caa agg cac acg acg aag cac ata ttg att      153
Gln Pro Asp Gly Ser Lys Gln Arg His Thr Thr Lys His Ile Leu Ile
        20                  25                  30

gca act ggc agc cga gca act ctg gtc aac att cct gga aag gag ctg      201
Ala Thr Gly Ser Arg Ala Thr Leu Val Asn Ile Pro Gly Lys Glu Leu
    35                  40                  45

gct att act tca gat gaa gct tta agt ttg gag gag cta ccg aaa cgt      249
Ala Ile Thr Ser Asp Glu Ala Leu Ser Leu Glu Glu Leu Pro Lys Arg
50                  55                  60                  65

gct gtg att ctt ggt ggc gga tat att gct gtt gaa ttt gct tct atc      297
Ala Val Ile Leu Gly Gly Gly Tyr Ile Ala Val Glu Phe Ala Ser Ile
                70                  75                  80

tgg aag ggg ttg ggt gct caa gta gat cta ttt tat agg aaa gaa ctt      345

Trp Lys Gly Leu Gly Ala Gln Val Asp Leu Phe Tyr Arg Lys Glu Leu
            85                  90                  95

cct cta agg ggt ttt gat gat gag atg aga aca gtt gtt gca agt aac      393
Pro Leu Arg Gly Phe Asp Asp Glu Met Arg Thr Val Val Ala Ser Asn
        100                 105                 110

ctt gaa gga agg gga atc cga ttg cat cca ggg aca aat ttg act gag      441
Leu Glu Gly Arg Gly Ile Arg Leu His Pro Gly Thr Asn Leu Thr Glu
    115                 120                 125

ttg agt aaa aca gcc gat ggc att aaa gtt gtg aca gat aag ggg gat      489
Leu Ser Lys Thr Ala Asp Gly Ile Lys Val Val Thr Asp Lys Gly Asp
130                 135                 140                 145

gag ttc att gca gat gtt gtt ctt ttt gct aca ggt cgt gcg ccg aac      537
Glu Phe Ile Ala Asp Val Val Leu Phe Ala Thr Gly Arg Ala Pro Asn
                150                 155                 160

tcc aat agg ctg aac ttg gaa gct gta ggt gtt gag gtt gat caa ata      585
Ser Asn Arg Leu Asn Leu Glu Ala Val Gly Val Glu Val Asp Gln Ile
            165                 170                 175

gga gcc atc aag gtt gat gaa tac tcc cgt aca tca gtt cca aat ata      633
Gly Ala Ile Lys Val Asp Glu Tyr Ser Arg Thr Ser Val Pro Asn Ile
        180                 185                 190

tgg gct gtt ggc gat gtg acg aat cgg att aac cta aca cct gtt gct      681
Trp Ala Val Gly Asp Val Thr Asn Arg Ile Asn Leu Thr Pro Val Ala
    195                 200                 205

ctg atg gag gct act tgc ttt gct aaa act gtg ttt ggt ggc cag aca      729
Leu Met Glu Ala Thr Cys Phe Ala Lys Thr Val Phe Gly Gly Gln Thr
210                 215                 220                 225

gtt aag cct gat tat aaa gat gta cct tgt gct gtt ttc tgt att ccg      777
Val Lys Pro Asp Tyr Lys Asp Val Pro Cys Ala Val Phe Cys Ile Pro
                230                 235                 240

ccg cta tct gtc gtg ggt ctt agc gaa caa gag gct ttg gcg gaa gcc      825
Pro Leu Ser Val Val Gly Leu Ser Glu Gln Glu Ala Leu Ala Glu Ala
            245                 250                 255

aag aat gat ctt ctt gtt tac aca tca agt ttc aat cca atg aag aac      873
Lys Asn Asp Leu Leu Val Tyr Thr Ser Ser Phe Asn Pro Met Lys Asn
        260                 265                 270

agc ata tcc aaa cgt caa gag aag tct atc atg aaa ctg gtg gtt gat      921
Ser Ile Ser Lys Arg Gln Glu Lys Ser Ile Met Lys Leu Val Val Asp
    275                 280                 285

gct gag acc gat aag gta ctc ggt gca gcc atg tgt gga cca gat gca      969
Ala Glu Thr Asp Lys Val Leu Gly Ala Ala Met Cys Gly Pro Asp Ala
290                 295                 300                 305

gct gaa att atg cag ggc att gct gtt gcg ctc aag gct gga gct acc     1017
Ala Glu Ile Met Gln Gly Ile Ala Val Ala Leu Lys Ala Gly Ala Thr
                310                 315                 320

aag gcg acc ttc gac agc act gtt ggg att cac ccc tct gcc gcg gag     1065
Lys Ala Thr Phe Asp Ser Thr Val Gly Ile His Pro Ser Ala Ala Glu

            325                 330                 335

gag ttt gtg acg atg cgg acc ttg acc agg cgc gtg agc ccg gca tcg 1113
Glu Phe Val Thr Met Arg Thr Leu Thr Arg Arg Val Ser Pro Ala Ser
        340                 345                 350

aag cca aag acg aac ttg taagcaagat ccctgcaatg ttttggacga 1161
Lys Pro Lys Thr Asn Leu
    355

aaattatact ggtgcggcga cgggaactgt agacgtggac tcttgtttca gttcagctag 1221

ccctgcgctt ggcatttgac ctgaaagggt gatacggtgt acgtatttga ttgtgatagt 1281

aataagtttc tgtggaacag tttgtacaga aatcgtgggt attgttgtga tttccaagcc 1341

aaaaaaaaaa aaaaaa 1357

<210> 4
<211> 1202
<212> DNA
<213> Hordeum vulgare L. cv. Ehimehadaka no.1

<400> 4
ggaaaggagc tggctattac ttcagatgaa gctttaagtt tggaggagct accgaaacgt 60

gctgtgattc ttggtggcgg atatattgct gttgaatttg cttctatctg gaaggggttg 120

ggtgctcaag tagatctatt ttataggaaa gaacttcctc taagggtttt tgatgatgag 180

atg aga aca gtt gtt gca agt aac ctt gaa gga agg gga atc cga ttg 228
Met Arg Thr Val Val Ala Ser Asn Leu Glu Gly Arg Gly Ile Arg Leu
1               5                   10                  15

cat cca ggg aca aat ttg act gag ttg agt aaa aca gcc gat ggc att 276
His Pro Gly Thr Asn Leu Thr Glu Leu Ser Lys Thr Ala Asp Gly Ile
            20                  25                  30

aaa gtt gtg aca gat aag ggg gat gag ttc att gca gat gtt gtt ctt 324
Lys Val Val Thr Asp Lys Gly Asp Glu Phe Ile Ala Asp Val Val Leu
        35                  40                  45

ttt gct aca ggt cgt gcg ccg aac tcc aat agg ctg aac ttg gaa gct 372
Phe Ala Thr Gly Arg Ala Pro Asn Ser Asn Arg Leu Asn Leu Glu Ala
    50                  55                  60

gta ggt gtt gag gtt gat caa ata gga gcc atc aag gtt gat gaa tac 420
Val Gly Val Glu Val Asp Gln Ile Gly Ala Ile Lys Val Asp Glu Tyr
65                  70                  75                  80

tcc cgt aca tca gtt cca aat ata tgg gct gtt ggc gat gtg acg aat 468
Ser Arg Thr Ser Val Pro Asn Ile Trp Ala Val Gly Asp Val Thr Asn
                85                  90                  95

cgg att aac cta aca cct gtt gct ctg atg gag gct act tgc ttt gct 516
Arg Ile Asn Leu Thr Pro Val Ala Leu Met Glu Ala Thr Cys Phe Ala
            100                 105                 110

aaa act gtg ttt ggt ggc cag aca gtt aag cct gat tat aaa gat gta 564

Lys Thr Val Phe Gly Gly Gln Thr Val Lys Pro Asp Tyr Lys Asp Val
        115                 120                 125

cct tgt gct gtt ttc tgt att ccg ccg cta tct gtc gtg ggt ctt agc     612
Pro Cys Ala Val Phe Cys Ile Pro Pro Leu Ser Val Val Gly Leu Ser
    130                 135                 140

gaa caa gag gct ttg gcg gaa gcc aag aat gat ctt ctt gtt tac aca     660
Glu Gln Glu Ala Leu Ala Glu Ala Lys Asn Asp Leu Leu Val Tyr Thr
145                 150                 155                 160

tca agt ttc aat cca atg aag aac agc ata tcc aaa cgt caa gag aag     708
Ser Ser Phe Asn Pro Met Lys Asn Ser Ile Ser Lys Arg Gln Glu Lys
                165                 170                 175

tct atc atg aaa ctg gtg gtt gat gct gag acc gat aag gta ctc ggt     756
Ser Ile Met Lys Leu Val Val Asp Ala Glu Thr Asp Lys Val Leu Gly
            180                 185                 190

gca gcc atg tgt gga cca gat gca gct gaa att atg cag ggc att gct     804
Ala Ala Met Cys Gly Pro Asp Ala Ala Glu Ile Met Gln Gly Ile Ala
        195                 200                 205

gtt gcg ctc aag gct gga gct acc aag gcg acc ttc gac agc act gtt     852
Val Ala Leu Lys Ala Gly Ala Thr Lys Ala Thr Phe Asp Ser Thr Val
    210                 215                 220

ggg att cac ccc tct gcc gcg gag gag ttt gtg acg atg cgg acc ttg     900
Gly Ile His Pro Ser Ala Ala Glu Glu Phe Val Thr Met Arg Thr Leu
225                 230                 235                 240

acc agg cgc gtg agc ccg gca tcg aag cca aag acg aac ttg             942
Thr Arg Arg Val Ser Pro Ala Ser Lys Pro Lys Thr Asn Leu
                245                 250

taagcaagat ccctgcaatg ttttggacga aaattatact ggtgcggcga cgggaactgt   1002

agacgtggac tcttgtttca gttcgctagc cctgcgcttg gcatttgacc tgaaagggtg   1062

ataggtgtac gtatttgatt gtgatagtaa taagtttctg tggaacagtt tgtacagaaa   1120

tcgtgggtat tgttgtgatt tccaagccaa ataagccctt gatgttgaaa aaaaaaaaaa   1182

aaaaaaaaaa aaaaaaaaaa                                               1202

<210> 5
<211> 1289
<212> DNA
<213> Hordeum vulgare L. cv. Ehimehadaka no.1

<400> 5
   cccagccaga tggttcaaag caaaggcaca cgacgaagca catattgatt gcaactggca     60
   gccgagcaac tctggtcaac attcctggaa aggagctggc tattacttca gatgaagctt    120
   taagtttgga ggagctaccg aaacgtgctg tgattcttgg tggcggatat attgctgttg    180
   aatttgcttc tatctggaag gggttgggtg ctcaagtaga tctattttat aggaaagaac    240
   ttcctctaag gggttttgat gatgag atg aga aca gtt gtt gca agt aac ctt 293
   Met Arg Thr Val Val Ala Ser Asn Leu
   1 5
   gaa gga agg gga atc cga ttg cat cca ggg aca aat ttg act gag ttg 341
   Glu Gly Arg Gly Ile Arg Leu His Pro Gly Thr Asn Leu Thr Glu Leu
   10 15 20 25
   agt aaa aca gcc gat ggc att aaa gtt gtg aca gat aag ggg gat gag 389
   Ser Lys Thr Ala Asp Gly Ile Lys Val Val Thr Asp Lys Gly Asp Glu
   30 35 40
   ttc att gca gat gtt gtt ctt ttt gct aca ggt cgt gcg ccg aac tcc 437
   Phe Ile Ala Asp Val Val Leu Phe Ala Thr Gly Arg Ala Pro Asn Ser
   45 50 55
   aat agg ctg aac ttg gaa gct gta ggt gtt gag gtt gat caa ata gga 485
   Asn Arg Leu Asn Leu Glu Ala Val Gly Val Glu Val Asp Gln Ile Gly
   60 65 70
   gcc atc aag gtt gat gaa tac tcc cgt aca tca gtt cca aat ata tgg 533
   Ala Ile Lys Val Asp Glu Tyr Ser Arg Thr Ser Val Pro Asn Ile Trp
   75 80 85
   gct gtt ggc gat gtg acg aat cgg att aac cta aca cct gtt gct ctg 581
   Ala Val Gly Asp Val Thr Asn Arg Ile Asn Leu Thr Pro Val Ala Leu
   90 95 100 105
   atg gag gct act tgc ttt gct aaa act gtg ttt ggt ggc cag aca gtt 629
   Met Glu Ala Thr Cys Phe Ala Lys Thr Val Phe Gly Gly Gln Thr Val
   110 115 120
   aag cct gat tat aaa gat gta cct tgt gct gtt ttc tgt att ccg ccg 677
   Lys Pro Asp Tyr Lys Asp Val Pro Cys Ala Val Phe Cys Ile Pro Pro
   125 130 135
   cta tct gtc gtg ggt ctt agc gaa caa gag gct ttg gcg gaa gcc aag 725
   Leu Ser Val Val Gly Leu Ser Glu Gln Glu Ala Leu Ala Glu Ala Lys
   140 145 150
   aat gat ctt ctt gtt tac aca tca agt ttc aat cca atg aag aac agc 773
   Asn Asp Leu Leu Val Tyr Thr Ser Ser Phe Asn Pro Met Lys Asn Ser
   155 160 165
   ata tcc aaa cgt caa gag aag tct atc atg aaa ctg gtg gtt gat gct 821
   Ile Ser Lys Arg Gln Glu Lys Ser Ile Met Lys Leu Val Val Asp Ala
   170 175 180 185
   gag acc gat aag gta ctc ggt gca gcc atg tgt gga cca gat gca gct 869
   Glu Thr Asp Lys Val Leu Gly Ala Ala Met Cys Gly Pro Asp Ala Ala
   190 195 200
   gaa att atg cag ggc att gct gtt gcg ctc aag gct gga gct acc aag 917
   Glu Ile Met Gln Gly Ile Ala Val Ala Leu Lys Ala Gly Ala Thr Lys
   205 210 215
   gcg acc ttc gac agc act gtt ggg att cac ccc tct gcc gcg gag gag 965
   Ala Thr Phe Asp Ser Thr Val Gly Ile His Pro Ser Ala Ala Glu Glu
   220 225 230
   ttt gtg acg atg cgg acc ttg acc agg cgc gtg agc ccg gca tcg aag 1013
   Phe Val Thr Met Arg Thr Leu Thr Arg Arg Val Ser Pro Ala Ser Lys
       235                 240                 245
   cca aag acg aac ttg taagcaagat ccctgcaatg ttttggacga aaattatact     1068
   Pro Lys Thr Asn Leu
   250
   ggtgcggcga cgggaactgt agacgtggac tcttgtttca gttcgctagc cctgcgcttg   1128
   gcatttgacc tgaaagggtg atacggtgta cagtatttga ttgtgatagt aataagtttc   1188
   tgtggaacag tttgtacaga aatcgtgggt attgttgtga tttccaagcc aaataagccc   1248
   ttgatgttga aatgttttcc catttaaaaa aaaaaaaaaa a                       1289